# EUROPEAN PATENT SPECIFICATION

(11) **EP 1 454 615 B1**
(45) Date of publication and mention of the grant of the patent: **05.10.2005**
(21) Application number: 03004866.4
(22) Date of filing: 06.03.2003
(51) Int. Cl.: A61K 7/13

(54) **Composition for dyeing human hair**
Mittel zum Färben von menschlichen Haaren
Composition pour la coloration des cheveux humains

(43) Date of publication of application: 08.09.2004
(73) Proprietor: KPSS-Kao Professional Salon Services GmbH, 64297 Darmstadt (DE)
(72) Inventor: Nöcker, Bernd, Dr., 64372 Ober-Ramstadt (DE); Vega-Carrascal, Isabel, 47014 Valladolid (ES)

(56) References cited:
- EP-A- 1 022 014
- FR-A- 762 985
- US-A- 4 182 612
- US-A- 5 601 620
- D.F. WILLIAMS: "Chemistry and Technology of the Cosmetics and Toiletries Industry" 1992 , BLACKIE ACADEMIC & PROFESSIONAL XP002251416 * page 92 - page 93 *

## Description

This invention relates to a hair dyeing composition showing excellent dyeing ability comprising at least one direct acting hair dye and N-(2-methoxybenzyl) urea. It should be well understood that the colouring compositions of this invention are ready to use compositions and therefore do not require any mixing with additional agents such as oxidizers prior to application.

Hair colouring is a common practice for ages. Oxidative colouration has been widely used for achieving durable, brilliant hair colour. Direct dyes, mainly of cationic character, have also found their applications for colouring hair. The colours so achieved are brilliant but often lacking durability. Recently, anionic direct dyes have been found to be very powerful for changing hair colour permanently and to achieve long lasting, brilliant colours. The colouring agents with anionic dyes are so formulated that the optimum conditions are realised for achieving the highest dyestuff penetration into hair. European patent application with laid open number EP 1022014 describes such compositions comprising anionic dyestuffs, solvents, as aid to enhance penetration of said dyestuffs, and a buffer solution to adjust the pH of the dyeing agent in the range from 2 to 6. For enhancing penetration of dyestuffs, solvents are used such as ethanol, benzyl alcohol, propylene carbonate, dipropylene glycol. Products are found on the professional hair dressing market applying this technology.

US 5,601,620, as well, discloses hair colouring agents with acid dyes, an organic solvent and at least one polysiloxane as a conditioner. The dyeing compositions disclosed here are having a pH in the range of 1.5 - 4.5. In this patent again ethanol, benzyl alcohol and dipropylen glycol are mentioned to be the penetration enhancers preferred.

FR-A-762985 discloses colouring compositions comprising urea and its derivatives. The documents is however silenton methoxy benzyl urea.

In practice further need of higher dyeing ability is obviously desired by professional hair dressing practitioners and also end consumers in order to achieve more brilliant and long lasting (fastness against washing) colorations.

Recently, it has surprisingly been found out that hair colouring compositions on aqueous basis and direct acting anionic dyes comprising, in addition to the conventional penetration enhancers, N-(2-methoxybenzyl) urea showing higher colouring ability than that of comprising only conventional solvents as penetration enhancers. Although the underlying mechanism of such effect is not very well understood, speculatively it may be observed because of synergistic interaction of N-(2-methoxybenzyl) urea and the penetration enhancer contained in the colouring compositions. This invention is explicitly suitable for colouring hair with direct acting dyestuffs and therefore, the compositions are free from any oxidation dye precursors and/or coupling agents.

Ready to use colouring compositions according to the invention comprises N-(2-methoxybenzyl) urea, at least one direct acting anionic hair dye, and at least are organic solvent, which is applied on to hair without any mixing with additional agents.

Concentration of N-(2-methoxybenzyl) urea in the colouring compositions of the present invention should be between 0.1 - 10%, preferably 0.1 - 7%, more preferably 0.2 - 5% and most preferably 0.2 - 3% by weight, calculated to the total composition.

According to the invention the suitable direct acting dyes are acidic, anionic dyes. Those are customarily incorporated in an amount from about 0.001 % to about 5 %, preferably about 0.01 % to about 2.5 %, in particular about 0.05 % to about 2.5 % by weight, calculated to the total composition, into the colouring compositions.

Examples for suitable anionic dyestuffs are:

| | |
|---|---|
| Acid Black 1, | C.I.-No. 20,470; |
| Acid Blue 1, | C.I.-No. 42,045; |
| Food Blue 5, | C.I.-No. 42,051; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.I.-No. 45,380; |
| Acid Red 92, | C.I.-No. 45,410; |
| Acid Orange 7, | C.I.-No. 15,510; |
| Acid Violet 43, | C.I.-No. 60,730; |
| Acid Yellow 1, | C.I.-No. 10,316; |
| Acid Yellow 23, | C.I.-No. 19,140; |
| Acid Yellow 3, | C.I.-No. 47,005; |
| Food Yellow No. 8, | C.I.-No. 14,270; |
| D&C Brown No. 1, | C.I.-No. 20,170 |
| D&C Green No. 5, | C.I.-No. 61,570; |
| D&C Orange No. 4, | C.I.-No. 15,510; |
| D&C Orange No. 10, | C.I.-No 45,425:1; |
| D&C Orange No. 11, | C.I.-No. 45,425; |
| D&C Red No. 21, | C.I.-No. 45,380:2; |
| D&C Red No. 27, | C.I.-No. 45,410:1; |
| D&C Red No. 33, | C.I.-No. 17,200; |
| D&C Yellow No. 7, | C.I.-No. 45,350:1; |
| D&C Yellow No. 8, | C.I.-No. 45,350; |
| FD&C Red No. 4, | C.I.-No. 14,700; |
| FD&C Yellow No. 6, | C.I.-No. 15,985. |

Direct acting cationic dyes can also be incorporated into the colouring compositions at a concentration less than 0.5%, preferably less than 0.25% by weight calculated to the total concentration of the composition. It should be kept in mind that those are less suited for the colouring compositions described here. Examples to those cationic dyes are:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 12 | C.I.-No. 48,070; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57. | C.I.-No. 12,719. |

Plant dyestuffs can also be used alone or in combination with synthetic direct-acting dyestuffs, for example henna (red or black), alkanna root, laccaic acid, indigo, logwood powder, madder root and rhubarb powder, etc.

It is self understood that the compositions according to the present invention comprise organic solvents as a penetration enhancers and also as a solubilizers for especially direct acting dyes. Examples of such "penetration enhancers" are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. The compositions can also contain benzyl urea and/or benzyl carbamate as a penetration enhancer as well. Typically the concentration of those solvents and/or penetration enhancer can be in the range from 1% to 50%, preferably 1 - 40%, more preferably 1 - 30% and most preferably 1 - 25% by weight calculated to the total composition.

The pH of such compositions is in the range of 1.0 - 5.0, preferably 1.5 to 4.5, more preferably 2 to 4 and most preferably 2 - 3.5. The pH of the compositions is adjusted to the desired value with an organic and/or inorganic or their mixtures. Suitable organic acids are such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid and inorganic acids are such as hydrochloric acid, phosphoric acid, sulphuric acid, nitric acid. Concentration of the organic and/or inorganic acids or their mixtures should be adjusted in a way that colouring composition so obtained has a pH value between 1.0 - 5.0, preferably 1.5 to 4.5, more preferably 2 to 4 and most preferably 2 - 3.5. Typically concentration for acids can be 0.2 - 30% by weight, preferably 0.5 - 15% by weight, more preferably 0.5 - 8% by weight and most preferably 0.5 - 6% by weight. The pH of the colouring composition can also be adjusted to the required pH by using alkaline solution such as sodium hydroxide, potassium hydroxide or their salts with those acids mentioned above in the case that at the selected acid concentration pH of the composition is lower than that of the aimed value.

The hair dyeing compositions according to the invention preferably also contain thickening agents. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof, synthetic polymers such as the various polyacrylic acids, etc., in amounts from about 0.3 % to about 5 % by weight, calculated to the total composition and depending on the desired consistency thereof.

Optionally, the colouring composition of this invention comprises hair conditioning agents. Conditioning agents can be selected from oily substances, non-ionic substances, cationic amphiphilic ingredients, cationic polymers or their mixtures. Oily substances are selected from such as silicone oils either volatile or non-volatile, natural and synthetic oils. Among silicone oils those can be added to the pretreatment composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II, respectively,

R₁ CO (O CH₂ CH₂)ₙ OH formula I

R₁ CO (O CH₂ CH₂)ₙ O OC R₂ formula II

where R₁ and R₂ are independent from each other saturated, unsaturated or branched or non-branched alkyl chain with 7 to 21 C atoms and n is typically 2 - 100.

Colouring composition can contain cationic amphiphilic conditioning ingredients according to the formula III, but not limited to. 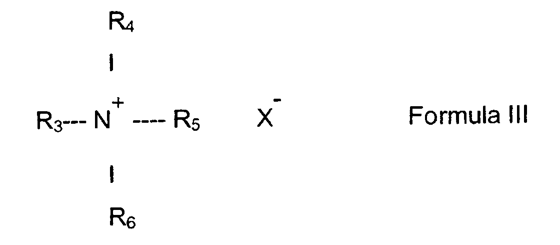 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 1 - 4 or

R₈ CO O (CH₂)ₙ

where R₈ is saturated or unsaturated, branched or non-branched alkyl chain with 7-21 C atoms and n has typical value of 1 - 4, and
R₄ is H or unsaturated or saturated, branched or non-branched alkyl chain with 1 - 22 C atoms or

R₇ CO NH (CH₂)ₙ

or

R₈ CO O (CH₂)n

where R₇, R₈ and n are same as above.

R₅ and R₆ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonium chloride, dioleoylethyl dimethyl ammonium methosulfate, dioleoylethyl hydroxyethylmonium methosulfate.

Colouring composition can also contain cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Typical concentration range for any of the conditioners mentioned above can be 0.01 - 5% by weight, preferably 0.03 - 2% by weight and more preferably 0.05 - 0.75% by weight.

The hair dyeing composition according to the invention can also comprise surface-active substances, although it does not constitute a tinting shampoo.

These can be non-ionic and/or amphoteric or zwitterionic.

Preferred are nonionic surfactants in an amount ranging between about 0.5 % and about 5 %, calculated to the total composition.

Suitable nonionic surfactants are compounds from the category of alkyl polyglucosides with the general formula

R₉-O-(CH₂CH₂O)ₙ-Zₓ,

wherein R₉ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates.
Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Further additionally useful surfactants are amineoxides.
Such amineoxides are state of the art, for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain.

Suitable amineoxides are on the market, for example, under the trade names "Ammonyx®", "Aromox®" or "Genaminox®".

Further optional surfactant components are fatty acid mono- and dialkanolamides, such as coco fatty acid monoethanolamide and myristic fatty acid monoisopropanolamide.

Suitable amphoteric or zwitterionic surfactants are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 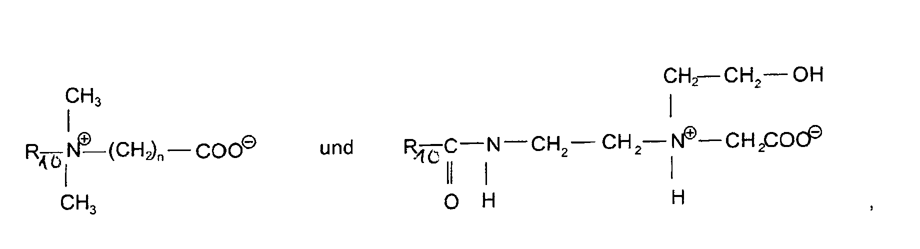 wherein R₁₀ is a C₈-C₁₈-alkyl group and n is 1 to 3,
sulfobetaines of the structure 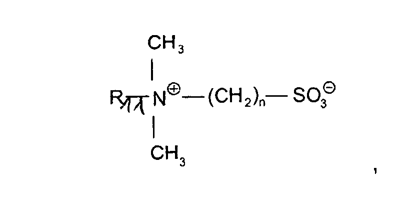 wherein R₁₁ is a C₈-C₁₈-alkyl group and n is 1 to 3,
and amidoalkyl betaines of the structure 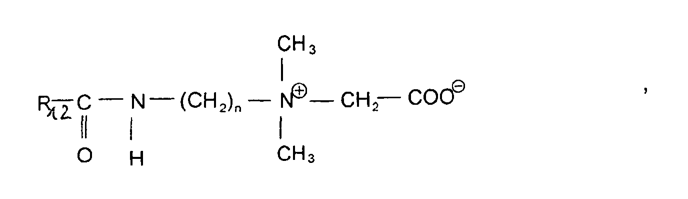 wherein R₁₂ is a C₈-C₁₈-alkyl group and n is 1 to 3,

Preferred are fatty acid amidoalkyl betaines, in particular cocoamidopropyl betaine, and cocoamphoacetate and -propionate, in particular the sodium salts thereof.

Colouring compositions of the present invention can be in the form of gel or emulsion. In the case of gel type of preparations the colouring compositions contain polymeric thickeners as mentioned above. In the case that colouring compositions are in emulsion form, the compositions may then contain fatty alcohols with 12 to 22 carbon atoms and non-ionic emulsifier such as ethoxylated fatty alcohols with general formula (IV)

R₁₃ (O CH₂ CH₂)ₙ OH formula IV

where R₁₃ is saturated or unsaturated, branched or non-branched alkyl chain with 8-22 C atoms and n has typical value of 2 - 100.

The viscosity of the compositions according to the invention preferably ranges from about 1,000 to about 60,000, in particular about 2,000 to 50,000, especially about 5,000 to 40,000 mPa.s, measured at 20°C with a Brookfield rotation viscosimeter, with spindle 5 at 5 rpm.

The invention is illustrated with the following examples but not limited to.

### Example 1

| | **A** | **B** |
|---|---|---|
| Acid red 33 | 0.5 | 0.5 |
| Ethanol | 15.00 | 15.00 |
| N-(2-methoxybenzyl) urea | 1.70 | - |
| PVM/MA Decadine Cross polymer | 2.50 | 2.50 |
| Sodium hydroxide | 0.25 | 0.25 |
| Lactic acid | 4.50 | 4.50 |
| Dimethicone copolyol | 1.00 | 1.00 |
| Water | to 100 | to 100 |
| | | |
| pH | 3.0 | 3.0 |

The viscosity of the compositions is 30,000 mPa.s measured at 20°C with a Brookfield rotation viscosimeter, with spindle 5 at 5 rpm.

An intensive magenta colour is achieved on goat tress with composition A. With composition B the colour is less intensive

### Example 2

| | **A** | **B** |
|---|---|---|
| Acid violet 43 | 0.3 | 0.3 |
| Ethanol | 15.00 | 15.00 |
| N-(2-methoxybenzyl) urea | 1.70 | - |
| PVM/MA Decadine Cross polymer | 2.50 | 2.50 |
| Sodium hydroxide | 0.25 | 0.25 |
| Lactic acid | 4.50 | 4.50 |
| Dimethicone copolyol | 1.00 | 1.00 |
| Water | to 100 | to 100 |
| pH | 3.0 | 3.0 |

The viscosity of the compositions is 30,000 mPa.s measured at 20°C with a Brookfield rotation viscosimeter, with spindle 5 at 5 rpm.

A dark violet colour is achieved on goat tress with composition A. With composition B the colour is less intensive

For both examples 1 and 2, preparations A are examples to illustrate the invention. Preparations B are comparative preparations and not illustrating the invention

The colouring compositions in examples 1 and 2 are prepared by dissolving firstly N-(2-methoxybenzyl) urea, if present, in ethanol-water mixture at around 55°C. Into the solution, polymer and the remaining raw materials are added and mixed until homogeneity.

Colouring test is carried out with both preparations using a goat hair tress. Amount of colouring composition applied is approximately 1 g / g goat hair and coloruing is achieved by processing at 50°C for 20 min. Afterwards the tresses are rinsed with water and shampooed once. After air drying, colour measurements are carried out with a commercial equipment (Minolta CR-200) in order to determine the difference in colour nature and intensity. Delta E values are calculated from the L, a and b values measured before and after colouration. Results are presented in Table I.

**Table I**

| | | **Delta E** |
|---|---|---|
| **Example 1** | A (invention) | 70.3 |
| | B (non-inventive) | 52.7 |
| | | |
| **Example 2** | A (invention) | 64.2 |
| | B (non-inventive) | 44.7 |

It should be noted that the higher the delta E value the stronger the difference. It is very obvious from the results presented in Table that the compositions comprising N-(2-methoxybenzyl) urea (compositions A) are showing higher colouring ability that those of without (compositions B).

Similar results are obtained with the examples below. Examples 3 to 5 are prepared in the same way as described for Examples 1 and 2 above.

### Example 3

| | |
|---|---|
| Acid Violet 43 | 0.30 |
| Acid black 1 | 0.30 |
| DC Orange 4 | 0.20 |
| Acid red 33 | 0.20 |
| Ethanol | 15.00 |
| N-(2-methoxybenzyl) urea | 1.70 |
| PVM/MA Decadine Crosspolymer | 2.50 |
| Sodium hydroxide | 0.25 |
| Lactic acid | 4.50 |
| Dimethicone copolyol | 1.00 |
| Water | to 100 |
| | |
| pH | 3.0 |

The viscosity of the compositions is 28,500 mPa.s measured at 20°C with a Brookfield rotation viscosimeter, with spindle 5 at 5 rpm.

An intensive blue-black colour is achieved on goat tress. Exclusion of N-(2-methoxybenzyl) urea results in a less intensive colouration.

### Example 4

| | |
|---|---|
| Acid Violet 43 | 0.30 |
| Acid black 1 | 0.30 |
| DC Orange 4 | 0.20 |
| Acid red 33 | 0.20 |
| Benzyloxyethanol | 1.00 |
| Ethanol | 10.00 |
| N-(2-methoxybenzyl) urea | 1,00 |
| Benzyl urea | 0.70 |
| Hydroxyethylcellulose | 1.50 |
| Sodium hydroxide | 0.25 |
| Phosphoric acid to pH 2.5 | q.s. |
| Dimethicone copolyol | 1.00 |
| Water | to 100 |

The viscosity of the compositions is 20,000 mPa.s measured at 20°C with a Brookfield rotation viscosimeter, with spindle 5 at 5 rpm.

An intensive blue-black colour is achieved on goat tress. Exclusion of N-(2-methoxybenzyl) urea results in a less intensive colouration.

### Example 5

| | |
|---|---|
| Acid red 52 | 0.50 |
| DC Orange 4 | 0.25 |
| Benzyloxyethanol | 1.00 |
| Ethanol | 10.00 |
| Benzyl carbamate | 0.70 |
| N-(2-methoxybenzyl) urea | 1.00 |
| Hydroxyethylcellulose | 1.50 |
| Sodium hydroxide | 0.25 |
| Lactic acid | 4.00 |
| Phosphoric acid to pH 2.0 | q.s. |
| Dimethicone copolyol | 1.00 |
| Water | to 100 |

The viscosity of the compositions is 18,800 mPa.s measured at 20°C with a Brookfield rotation viscosimeter, with spindle 5 at 5 rpm.

An intensive red colour is achieved on goat tress. Exclusion of N-(2-methoxybenzyl) urea results in a less intensive colouration.

## Claims

1. Ready to use colouring composition for hair **characterized in that** it comprises N-(2-methoxybenzyl) urea at least one direct acting anionic dyestuff, and at least one organic solvent, which is applied on to hair without any mixing with additional agents.

2. Colouring composition for hair according to any of the preceeding claims **characterized in that** it comprises N-(2-methoxybenzyl) urea at a concentration of 0.1 - 10% by weight, calculated to the total composition.

3. Colouring composition for hair according to an of the preceeding claims **characterized in that** it comprises direct acting anionic dyestuffs at a concentration of 0.001 - 5% by weight calculated to the total composition.

4. Colouring composition for hair according to any of the preceeding claims **characterized in that** it further comprises benzyl urea and/or benzyl carbamate.

5. Colouring composition for hair according to any of the preceeding claims **characterized in that** the pH of the composition is in the range from 1 to 5.

6. Colouring composition for hair according to any of the preceeding claims **characterized in that** it further comprises organic or inorganic acids or their mixtures.

7. Colouring composition for hair according to any of the preceeding claims **characterized in that** it further comprises at least one physiologically compatible hair conditioning agent selected from oily substances, non-ionic substances, cationic amphiphilic compounds, cationic polymers or their mixtures.

8. Colouring composition for hair according to any of the preceeding claims **characterized in that** it further comprises polymeric thickening agents.

9. Colouring composition for hair according to any of the preceding claims **characterised in that** it has viscosity from 1,000 mPa.s to 60,000 mPa.s measured at 20°C with Brookfield viscosimeter with for example Spindle 5 at 5 rpm.

## Patentansprüche

1. Gebrauchsfertiges Haarfärbemittel, **dadurch gekennzeichnet, dass** es N-(2-methoxybenzyl)-urea, mindestens einen direktziehenden, anionischen Farbstoff und mindestens ein organisches Lösungsmittel enthält, und ohne Mischen mit zusätzlichen Substanzen auf das Haar aufgetragen wird.

2. Haarfärbemittel nach Anspruch 1, **dadurch gekennzeichnet, dass** es N-(2-methoxybenzyl)-urea in einer Menge von 0,1 bis 10 Gew.-%, berechnet auf die Gesamtzusammensetzung, enthält.

3. Haarfärbemittel nach Anspruch 1 und 2, **dadurch gekennzeichnet, dass** es anionische, direktziehende Farbstoffe in einer Menge von 0,001 bis 5 Gew.-%, berechnet auf die Gesamtzusammensetzung, enthält.

4. Haarfärbemittel nach einem der Ansprüche 1 bis 3, **dadurch gekennzeichnet, dass** es zusätzlich Benzylurea und/oder Benzylcarbamat enthält.

5. Haarfärbemittel nach einem der Ansprüche 1 bis 4, **dadurch gekennzeichnet, dass** es einen pH-Wert im Bereich von 1 bis 5 hat.

6. Haarfärbemittel nach einem der Ansprüche 1 bis 5, **dadurch gekennzeichnet, dass** es auch organische- oder anorganische Säuren oder deren Mischungen enthält.

7. Haarfärbemittel nach einem der Ansprüche 1 bis 6, **dadurch gekennzeichnet, dass** es zusätzlich mindestens eine physiologisch verträgliche Haarpflegesubstanz, ausgewählt aus öligen, nichtionischen, kationisch amphiligischen Substanzen, kationischen Polymeren oder deren Mischungen, enthält.

8. Haarfärbemittel nach einem der Ansprüche 1 bis 7, **dadurch gekennzeichnet, dass** es polymerische Verdickungsmittel enthält

9. Haarfärbemittel nach einem der Ansprüche 1 bis 8, **dadurch gekennzeichnet, dass** es eine Viskosität von 1000 bis 60000 mPa.s, gemessen bei 20°C mit einem Brookfield Viskosimeter; mit einer Spindel 5 bei 5 rpm, hat.

## Revendications

1. Composition colorante prête à l'emploi pour les cheveux **caractérisée en ce qu'**elle comprend de la N-(2-méthoxybenzyl)urée, au moins une matière colorante anionique à action directe et au moins un solvant organique, qui est appliquée sur les cheveux sans mélange avec des agents supplémentaires.

2. Composition colorante pour les cheveux selon l'une quelconque des revendications précédentes, **caractérisée en ce qu'**elle comprend de la N-(2-méthoxybenzyl)urée à une concentration de 0,1 à 10 % en poids, rapportée à la composition totale.

3. Composition colorante pour les cheveux selon l'une quelconque des revendications précédentes, **caractérisée en ce qu'**elle comprend des matières colorantes anioniques à action directe à une concentration de 0,001 à 5 % en poids, rapportée à la composition totale.

4. Composition colorante pour les cheveux selon l'une quelconque des revendications précédentes, **caractérisée en ce qu'**elle comprend en outre de la benzylurée et/ou du carbamate de benzyle.

5. Composition colorante pour les cheveux selon l'une quelconque des revendications précédentes, **caractérisée en ce que** le pH de la composition est dans la gamme de 1 à 5.

6. Composition colorante pour les cheveux selon l'une quelconque des revendications précédentes, **caractérisée en ce qu'**elle comprend en outre des acides organiques ou minéraux ou leurs mélanges.

7. Composition colorante pour les cheveux selon l'une quelconque des revendications précédentes, **caractérisée en ce qu'**elle comprend en outre au moins un agent de conditionnement des cheveux compatible au plan physiologique choisi parmi les substances huileuses, les substances non ioniques, les composés amphiphiles cationiques, les polymères cationiques et leurs mélanges.

8. Composition colorante pour les cheveux selon l'une quelconque des revendications précédentes, **caractérisée en ce qu'**elle comprend en outre des agents épaississants polymères.

9. Composition colorante pour les cheveux selon l'une quelconque des revendications précédentes, **caractérisée en ce qu'**elle a une viscosité de 1 000 mPa.s à 60 000 mPa.s, mesurée à 20°C avec un viscosimètre Brookfield avec, par exemple, une broche N° 5 à 5 tours par minute.
